## Europäisches Patentamt

## European Patent Office

## Office européen des brevets

(19)

(11) Veröffentlichungsnummer: **0 275 825**
**B1**

(12)

# EUROPÄISCHE PATENTSCHRIFT

(45) Veröffentlichungstag der Patentschrift:
**26.09.90**

(51) Int. Cl.⁵: **G01N 33/44,** G01N 3/10

(21) Anmeldenummer: **87810738.2**

(22) Anmeldetag: **10.12.87**

(54) Verfahren zur Messung von Volumenänderungen von Flüssigkeiten, insbesondere Schwundmessungen an Kunststoffen während der Härtung und Vorrichtung zur Ausführung des Verfahrens.

(30) Priorität: **16.12.86 CH 4994/86**

(43) Veröffentlichungstag der Anmeldung:
**27.07.88 Patentblatt 88/30**

(45) Bekanntmachung des Hinweises auf die Patenterteilung:
**26.09.90 Patentblatt 90/39**

(84) Benannte Vertragsstaaten:
**AT CH DE FR GB IT LI NL**

(56) Entgegenhaltungen:
**FR-A- 1 316 591**
**FR-A- 2 377 042**
**GB-A- 1 275 262**
**US-A- 4 069 703**

(73) Patentinhaber: **CIBA-GEIGY AG, Klybeckstrasse 141, CH-4002 Basel(CH)**

(72) Erfinder: **Poll, Dietmar, Neumatt 254, CH-1711 St. Silvester(CH)**

ACTORUM AG

## Beschreibung

Bei der Anwendung von Kunststoffen sind eine Vielzahl von Materialeigenschaften und Verhaltensweisen während verschiedener Phasen der Verarbeitung zu messen und zu steuern. Die vorliegende Erfindung bezieht sich insbesondere auf die Aufklärung von Vorgängen während der Formfüll- und Härtungsphase bei duroplastischen Kunststoffen und innerhalb dieses Problemkreises mit der Aenderung der Dichte während einer chemischen Reaktion, Schwund genannt.

Es sind eine Anzahl von Schwundmessverfahren bekannt, so die Auftriebsmethode, wobei die Messung des Auftriebes im allgemeinen in einem beheizten Oelbad durchgeführt wird. Bei bekannter thermischer Ausdehnung des Oels und nach Messung des Tara- und Bruttoauftriebes lässt sich die Dichte, bzw. das spezifische Volumen der Probe bei isothermen Bedingungen berechnen. Bei nicht isothermen Bedingungen erhält man jedcch keine gültigen Werte. Es sind auch Verfahren bekannt, bei welchen die Geometrieänderungen an Formkörpern gemessen werden, beispielsweise Längen- oder Volumenänderungen, doch auch hier wird der Schwund nur in der festen Phase unter isothermen Bedingungen festgestellt, während der Temperaturverlauf nicht mitgemessen werden kann. Auch den übrigen Verfahren bei der Messung von Längenänderungen haften verschiedene Nachteile an, wie die fehlende Temperaturkontrolle und -konstanz, der Einfluss der Geometrie solcher Giessteile auf die Verteilung des Schwundes in die drei Dimensionen, der nicht messbare Schwund in flüssiger Phase und der Einfluss von Formtrennmitteln. Des weiteren kann für ganz bestimmte Anwendungen der Schwund aus der Messung einer Verformung des Substrates, auf welchem die zu messende Schicht aufgebracht worden ist, ermittelt werden, doch auch bei diesem Verfahren kann der Schwund nicht in der flüssigen Phase gemessen werden. Ausserdem gibt es noch Verfahren mit Hilfe der Messung der Aenderung des elektrischen Widerstandes, die jedoch auch nicht vollständige Angaben über den Schwund geben.

Es ist demgegenüber Aufgabe der vorliegenden Erfindung, ein Verfahren zur Messung von Volumenänderungen an Kunststoffen während der Härtung anzugeben, mittels welchem insbesondere Schwundmessungen durchgeführt werden können, bei denen der Reaktionsablauf unter nicht-isothermen Bedingungen mit Erfassung der wichtigsten Parameter vor sich geht. Eine weitere Aufgabe ist es, eine Vorrichtung zur Ausführung dieses Verfahrens anzugeben, mit welcher auf relativ einfache Art Schwundmessungen über die ganze Reaktionsdauer durchgeführt und einer Recheneinheit eingegeben werden können. Ein Verfahren und eine Vorrichtung zur Ausführung dieses Verfahrens, die diese Aufgaben lösen, sind in den Ansprüchen angegeben.

In US-A-4.069.703, GB-A-1 275 262 und FR-A-1 316 591 sind sich weitestgehend ähnliche Apparaturen zur Relaxationsprüfung bereits fertig geformter Thermoplast-Proben in Form von Zugstäben oder Quadern beschrieben. Diese Proben werden in die beschriebenen Apparaturen erst nach einer gesondert erfolgten Herstellung eingespannt, mit Spannung bzw. Kraft unter Temperatureinfluss beaufschlagt und deren Längenänderung gemessen. Im Unterschied dazu kann bei der erfindungsgemässen Vorrichtung mit extrem niederviskosen reaktiven Flüssigkeiten ein Probekörper hergestellt werden und dessen Verlauf des Volumens während des Formgebungsprozesses unter Einfluss von Druck (Spannung) und Temperatur verfolgt werden. Aus diesen Beobachtungen kann man auf thermische Ausdehnung im flüssigen und festen Zustand und auf Schwund und Relaxationszeiten während des Härtungsvorganges schliessen.

Weiter ist in FR-A-2 377 042 eine Apparatur zur Verfolgung der Vernetzungsreaktion von extrem hochviskosen Elastomer-Proben in Tablettenform beschrieben. Dabei wird in ein 3,5 mm geöffnetes Werkzeug eine Tablette Kautschuk-Material gelegt und danach auf dieses Werkzeug ein Schliessdruck ausgeübt. Durch die Menge des austretenden Materials und des Forminnendruckes werden Rückschlüsse auf den Vernetzungsgrad gezogen. Die erfindungsgemässe Vorrichtung dagegen ist ein geschlossenes System, bei dem austretende Masse katastrophale Folgen auf die Messergebnisse hätte, da der Zusammenhang zwischen eingewogener Probenmenge und Volumen gestört würde.

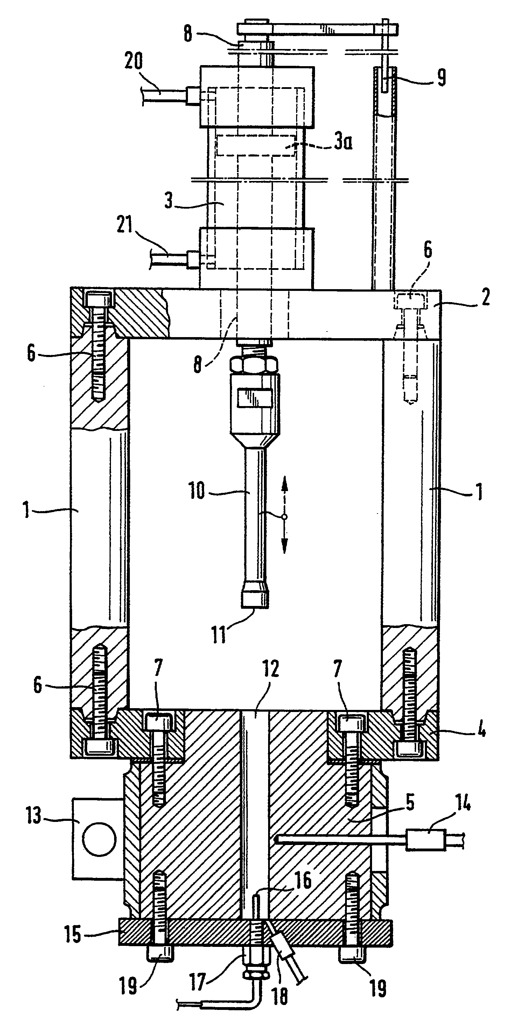

Die Erfindung wird im folgenden anhand einer Zeichnung eines Ausführungsbeispieles einer Messvorrichtung zur Messung von Volumenveränderungen näher erläutert, wobei die einzige Figur die erfindungsgemässe Vorrichtung zeigt.

Bei der Messung von Volumenänderungen an Kunststoffen, insbesondere Duroplasten, während der Aushärtungsphase, ist es notwendig, homogene Proben ohne Blasen, Bruchflächen und Schwundmarkierungen zu erhalten. Vorversuche mit einem bestimmten duroplastischen Harzsystem haben ergeben, dass dies bei Drücken von über 18 bar möglich ist. Es ist selbstverständlich, dass für andere Kunststoffe andere Zahlen gelten können. Wichtig ist daher, in Vorversuchen den Mindestdruck zu ermitteln, oberhalb welchem fehlerfreies Giessen möglich ist. Ausgehend von diesen Erkenntnissen wurde eine Vorrichtung konstruiert, die folgendem Anforderungsprofil genügt:

Messung von Druck, Temperatur im Formteil und Volumen direkt, gleichzeitig und unabhängig voneinander;

Möglichst kleines Probenvolumen;

Druckbereich 0-100 bar;

Temperaturbereich bis 250° C;

Einfache Handhabung, vor allem Reinigung;

Daten sollen mittels einer Rechenanlage verarbeitbar sein.

Eine Vorrichtung, die obigen Anforderungen genügt, ist in der Figur dargestellt. Die Vorrichtung weist ein Gestell mit vier Säulen 1 auf, an denen eine obere Trägerplatte 2 für den Druckzylinder 3 und eine untere Trägerplatte 4 für den Messzylinder 5 mittels Schrauben 6 befestigt sind. Der Messzylinder 5 ist mittels Schrauben 7 an der unteren Trägerplatte 4 befestigt. Die Kolbenstange 8 des Druckzylinders ist mit einem induktiven Wegaufnehmer 9 verbunden, womit der Weg des Messkolbens 10 sehr genau gemessen werden kann. In vorliegender Vorrichtung beträgt das Verhältnis der Flächen des Kolbens 3a des Druckzylinders zur Messfläche 11 des Messzylinders 10 9,33:1, wodurch ein Uebersetzungsverhältnis des Forminnendruckes von 1:9,33 resultiert. Dieser Forminnendruck wird beim Niedergleiten des Kolbens in der Messbohrung 12 erzeugt. Der Messzylinder 5 kann über eine genau steuerbare elektrische Heizung 13 auf bestimmte Temperaturen gebracht werden. Diese Temperatur wird durch eine Messzylinder-Temperatursonde 14 bestimmt. Am Messzylinder 5 ist unten ein Deckel 15 angeflanscht, in welchem genau in der Mitte ein Thermoelement 16 zur Bestimmung der Temperatur im Probeninnern in einer Druckverschraubung 17 eingelassen ist. Das Thermoelement 16 muss derart weit in die Probe eingelassen werden, dass nur deren Temperatur aufgenommen wird, ohne direkte Beeinflussung durch die Temperatur des Messzylinders. In vorliegendem Beispiel, bei einem Bohrungsdurchmesser von 15 mm, ist das Thermometer in einer Tiefe von 23 mm, gemessen vom Ende des Messzylinders, eingelassen. Berechnungen haben gezeigt, dass in dieser Tiefe die Beeinflussung durch die nicht zur Probe gehörenden Teile vernachlässigbar klein ist. Im Deckel eingelassen und bündig mit dessen messzylinderseitigen Oberfläche befindet sich bei der Messbohrung 12 ein piezoelektrischer Druckaufnehmer 18. Der Deckel ist ferner mittels Schrauben 19 am Messzylinder angeschraubt.

Der Druckzylinder 3 wird über eine Pneumatikleitung mit Druck beaufschlagt, wobei hier nur deren Zuleitung 20 und deren Auslass 21 eingezeichnet sind. Der zugeführte Druck wird über ein Manometer in der Pneumatikleitung gemessen, woraus der Forminnendruck über das oben genannte Verhältnis berechnet werden kann. Zusätzlich kann der Druck über eine nicht eingezeichnete Pneumatiksteuerung wahlweise derart gesteuert werden, dass zwei verschiedene Druckpegel abwechselnd hintereinander beaufschlagt werden können.

Aus der Beschreibung der Apparatur mit den verschiedenen Sonden und Aufnehmern geht hervor, dass das spezifische Volumen, die Temperatur und der Druck der Probe sowie der Forminnendruck und die Temperatur des Messzylinders direkt, gleichzeitig und unabhängig voneinander und unter nicht-isothermen Bedingungen ermittelt werden können. Vorzugsweise sind sämtliche Sonden und Aufnehmer direkt mit einer hier nicht eingezeichneten Rechnereinheit verbunden, so dass die Messdaten kontinuierlich aufgenommen und verarbeitet werden können. Berechnungen zeigen, dass mit geeigneten Sonden und Aufnehmern ein maximaler Fehler im spezifischen Volumen von 0,0002 cm³/g nicht überschritten wird. Bei einer typischen Schwundmessung wurde die Messbohrung nach Temperierung der Messzylinders mit kalter evakuierter Harzmischung gefüllt, sofort verschlossen und mit Druck beaufschlagt. Etwa 3s nach der Befüllung konnte die Volumenmessung bereits gültige Werte liefern. Sofort darauf wurden bei mehreren Solltemperaturen von 60 - 112° C und Forminnendrücken von 9,3 bis 37,3 bar (entsprechend 1 bzw. 4 bar Luftdruck) Volumenveränderung und Probekörpertemperatur gemessen. Daraus konnte ein Temperaturprofil gewonnen werden, anhand dessen der Schwund berechnet wurde. Um ein Abschwinden des Probekörpers von der Zylinderwand nach der Gelierung zu verhindern, muss entweder ein gewisser Forminnendruck herrschen oder die Probe mit Silikonöl überschichtet werden. Es wurde gefunden, dass 37,3 bar Forminnendruck ausreichen, um ohne Oelüberschichtung auszukommen. Andernfalls muss die Probe zusätzlich mit etwa 1 cm³ Silikonöl überschichtet werden, was bei der Auswertung eine Korrekturgleichung des Oels bedingt, die jedoch mit dieser Vorrichtung leicht ermittelt werden kann.

Wie bereits eingangs erwähnt, sind nicht nur Schwundmessungen mit dieser Vorrichtung möglich, sondern auch andere Messungen über Volumenveränderungen. So kann beispielsweise mit Hilfe der Drucksteuerung, die zwei Druckpegel erlaubt, die Veränderung von Kompressionsmodul und Relaxationszeit festgestellt werden. Ausserdem ergibt sich die Möglichkeit, mit der neuen Vorrichtung das spezifische Volumen von Flüssigkeiten unter verschiedenen Drücken bis 100 bar (Kompressibilität) und Temperaturen bis 250° C (thermische Ausdehnung) an einer einzigen Probe zu bestimmen, und aus einer Messreihe noch gleichzeitig den Gasgehalt und die Kompressibilität der ideal gasfreien Flüssigkeit zu berechnen. Dabei wird der Effekt ausgenutzt, dass sich ein Gas wesentlich mehr komprimieren lässt als eine Flüssigkeit.

Die Bestimmung des Gasgehaltes von Flüssigkeiten wie z.B. Luftgehalt in Giess- oder Schaumharzen kann bei hohen Anteilen durch Vergleichen der Dichtebestimmung von gashaltigem und evakuiertem Harz im Pyknometer mit ausreichender Genauigkeit durchgeführt werden. Ist der Gasgehalt jedoch < 1%, was z.B. bei Elektrogiessharzen noch zur Beeinträchtigung der Endeigenschaften führt, müssen genauere Messverfahren angewendet werden. Die erfindungsgemässe Vorrichtung ermöglicht nun aufgrund der einfachen Bedienung und der sehr genauen Messung des spezifischen Volumens bei unterschiedlichen Drücken (Kompressibilität) eine Berechnung des Gasgehaltes und der Kompressibilität der ideal gasfreien Flüssigkeit.

Ausgehend von der Zustandsgleichung idealer Gase:

p . V = n . R . T = konstant bei isothermen Bedingungen kann man folgende Beziehung zwischen dem spe- · zifischen Volumen der ideal gasfreien Flüssigkeit $V_{sp,H}$;
der Kompressibilität der ideal gasfreien Flüssigkeit K;

und dem Volumenanteil des Gases bei 1 bar Normaldruck $V_G \left[ \dfrac{cm^3 \cdot 1 \, bar}{g \, Harz} \right]$ herstellen, und man erhält folgende Gleichung:

$$K \cdot V_{sp,H} = \frac{V_{s1} - V_{s2}}{p_2 - p_1} - \frac{V_G}{(p_1+1) \cdot (p_2+1)}$$

wobei $V_{s1...n}$ = spezifisches Volumen bei Drücken $1 ... n \, |bar|$;

$p_{1...n}$ = Forminnendrücke 1 ... n |bar| ist.

Aus dieser Gleichung lassen sich die gewünschten Werte, d.h. der Volumenanteil des Gases bei 1 bar Normaldruck sowie die Kompressibilität der ideal gasfreien Flüssigkeit berechnen.

Die beschriebene Vorrichtung ist nicht an die angegebenen Werte gebunden, so dass sowohl die Dimensionen der Messbohrung und des hineinpassenden Messkolbens als auch die Druckrelationen zwischen Druckkolbenfläche und Fläche des Messkolbens andere Werte aufweisen können. Ausserdem kann die Messung der Wegstrecke des Kolbens, bzw. der Kolbenstange 8 auf anderem als induktivem Wege, beispielsweise auf optischem Wege, erfolgen. Ausserdem kann die Temperatur des Druckzylinders 5 durch eine andere Vorrichtung als eine elektrische Heizung 13 verändert werden. Wichtig ist, dass die Messung des Weges des Messkolbens, des Druckes und der Temperaturen direkt und unabhängig voneinander erfolgt, so dass vor allem auch nicht- isotherme Bedingungen erfasst werden können.

### Patentansprüche

1. Verfahren zur Messung von Volumenänderungen an Kunststoffen während der Härtung, insbesondere Schwundmessungen, bei dem das auszumessende Material in der Messbohrung eines Messzylinders mit einem solchen Druck beaufschlagt wird, dass eine völlig homogene Probe ohne Riss- und Blasenbildungen erzielt wird und dass gleichzeitig und unabhängig voneinander unter nicht-isothermen Bedingungen der beaufschlagte Druck, der durch die Probe auf den Boden des Messzylinders ausgeübte Druck, die Temperatur in der Probe und in dem Messzylinder und das Volumen der Probe gemessen werden.

2. Verfahren nach Anspruch 1, bei dem der auf die Probe ausgeübte Druck, falls keine Oelbeschichtung der Probe verwendet wird, für ein duroplastisches Kunstharz mindestens 18 bar beträgt.

3. Verfahren nach Anspruch 1 oder 2, bei dem die Messwerte kontinuierlich, bei Kunstharzen insbesondere auch vor, während und nach der Gelierphase, aufgenommen und einer Recheneinheit zugeführt werden.

4. Verfahren nach einem der Ansprüche 1 bis 3, bei dem der Druck abwechslungsweise auf zwei verschiedenen Pegeln beaufschlagt wird, um eine Veränderung des Kompressionsmoduls und der Relaxations bzw. Retardationszeit während der Härtung des Kunststoffes zu messen.

5. Verfahren nach Anspruch 1, bei dem das spezifische Volumen von Flüssigkeiten unter verschiedenen Drücken und Temperaturen bestimmt wird und daraus der Gasgehalt und die Kompressibilität der ideal gasfreien Flüssigkeit berechnet wird.

6. Vorrichtung zur Messung von Volumenänderungen an Kunststoffen während der Härtung, insbesondere Schwundmessungen, welche Vorrichtung einen Messzylinder (5) mit einer Messbohrung (12) und einen in diese Messbohrung passenden, druckbeaufschlagten Messkolben (10) sowie einen dem Messkolben zugeordneten Wegaufnehmer (9), eine Temperatursonde (14) im Messzylinder, eine Temperatursonde (16) in der Messbohrung zur Messung der Temperatur in der Probe, einen Druckmesser in der Druckluftzufuhrleitung, einen Druckaufnehmer (18) an dem Messkolben entgegengesetzten Ende der Messbohrung und eine Heizung (13) für den Messzylinder aufweist.

7. Vorrichtung nach Anspruch 6, bei der die Temperatursonde (16) in der Messbohrung sowie der Druckaufnehmer (18) in einem am Messzylinder abnehmbar befestigten Deckel (15) eingelassen sind.

4

8. Vorrichtung nach Anspruch 6 oder 7, bei der der Messkolben (10) von einem Druckkolben (3a) beaufschlagt wird, dessen wirksame Fläche ein Vielfaches, vorzugsweise etwa ein Zehnfaches der wirksamen Fläche (11) des Messkolbens beträgt.

9. Vorrichtung nach einem der Ansprüche 6 bis 8, bei der ein aus vier Säulen (1) bestehendes Gestell mit einer oberen, den Druckzylinder (3) tragenden Platte (2) und einer unteren, den Messzylinder (5) tragenden Platte (4) aufweist.

10. Vorrichtung nach einem der Ansprüche 6 bis 9, bei der die Druckluftzufuhrleitung eine Steuerung aufweist, um die Druckluft abwechslungsweise auf zwei verschiedenen Pegeln zu beaufschlagen.

## Claims

1. A process for measuring the variations in volume in synthetic materials during hardening, especially shrinkage measurements, in which the material which is to be measured in the measuring bore of a measuring cylinder is acted upon by such a pressure that a completely homogenous test piece is obtained without formation of any fractures or blisters and that simultaneously and independently of one another, the pressure applied, the pressure exerted by the test piece on the base of the measuring cylinder, the temperature in the test piece and in the measuring cylinder and the volume of the test piece are measured under nonisothermal conditions.

2. A process according to Claim 1, in which the pressure exerted on the test piece, should no oil coating of the test piece be used, is at least 18 bar for a duroplastic synthetic resin.

3. A process according to Claim 1 or 2, in which the measured values are continuously detected, and in particular in the case of synthetic resins are also recorded before, during and after the gelling phase, and are transmitted to a computing unit.

4. A process according to one of Claims 1 to 3, in which the pressure acts in an alternating manner on two differing levels so as to measure a variation of the compression module and the relaxation or retardation time during the hardening of the synthetic material.

5. A process according to Claim 1, in which the specific volume of fluids is ascertained under varying pressures and temperatures, and from this the gas content and the compressibility of the ideally gas-free fluid is calculated.

6. A device for measuring the variations in volume in synthetic materials during hardening, especially shrinkage measurements, which device has a measuring cylinder (5) comprising a measuring bore (12) and a measuring piston (10) which fits into this measuring bore and is acted upon by pressure, as well as a displacement detector (9) attached to the measuring piston, a temperature probe (14) in the measuring cylinder, a temperature probe (16) in the measuring bore for measuring the temperature in the test piece, a pressure gauge in the compressed air line, a pressure receiver (18) located at the end of the measuring bore opposite to the measuring piston and a heating means (13) for the measuring cylinder.

7. A device according to Claim 6, in which the temperature probe (16) is introduced into the measuring bore and the pressure receiver (18) is introduced into a removable cover (15) secured to the measuring cylinder.

8. A device according to Claim 6 or 7, in which the measuring piston (10) is acted upon by a pressure piston (3a), the effective surface of which is a multiple, preferably about ten times, the effective surface (11) of the measuring piston.

9. A device according to one of Claims 6 to 8, in which a framework comprising four uprights (1) has an upper plate (2) bearing the pressure cylinder (3) and a lower plate (4) bearing the measuring cylinder (5).

10. A device according to one of Claims 6 to 9, in which the compressed air line has a control, so that the compressed air can be caused to act in an alternating manner at two differing levels.

## Revendications

1. Procédé de mesure de variations de volume sur des matières plastiques au cours de leur durcissement, en particulier de mesure de retrait, procédé dans lequel on applique à la matière à examiner, dans l'ouverture de mesure d'un cylindre de mesure, une pression qui donne un échantillon tout à fait homogène, sans fissures ni bulles ou soufflures, et on mesure simultanément et indépendamment les uns des autres, dans des conditions non-isothermes, la pression appliquée, la pression exercée par l'échantillon sur le fond du cylindre de mesure, la température dans l'échantillon et dans le cylindre de mesure et le volume de l'échantillon.

2. Procédé selon la revendication 1 dans lequel la pression exercée sur l'échantillon, dans le cas où celui-ci n'a pas été enduit d'une huile, est d'au moins 18 bar pour une résine duroplaste.

3. Procédé selon la revendication 1 ou 2 dans lequel on prend les mesures en continu, pour des résines synthétiques, en particulier également avant, pendant et après la phase de gélification, et on les transmet à une unité de calcul.

4. Procédé selon l'une des revendications 1 à 3 dans lequel la pression est appliquée alternativement à deux niveaux différents pour mesurer une variation du module de compression et du temps de relaxation ou retard au cours du durcissement de la matière plastique.

5. Procédé selon la revendication 1 dans lequel on détermine le volume spécifique de liquides sous diverses pressions et à diverses températures, d'après lequel on calcule la teneur en gaz et le coefficient de compressibilité du liquide théoriquement sans gaz.

6. Appareil de mesure de variations de volume sur des matières plastiques au cours de leur durcissement, en particulier de mesure de retrait, appareil qui comprend un cylindre de mesure (5) avec une ouverture de mesure (12) et un piston de mesure (10) mis sous pression qui passe dans l'ouverture de mesure, ainsi qu'un capteur de déplacement (9) conjugué au piston de mesure, une sonde de température (14) dans le cylindre de mesure, une sonde de température (16) dans l'ouverture de mesure pour mesurer la température dans l'échantillon, un mesureur de pression dans le conduit d'arrivée de l'air sous pression, un capteur de pression (18) à l'extrémité de l'ouverture de mesure opposée au piston de mesure et un chauffage (13) du cylindre de mesure.

7. Appareil selon la revendication 6 dans lequel la sonde de température (16) dans l'ouverture de mesure et le capteur de pression (18) sont introduits dans un couvercle (15) fixé au cylindre de mesure et démontable.

8. Appareil selon la revendication 6 ou 7 dans lequel le piston de mesure (10) est actionné par un piston de pression (3a) dont la surface efficace est de plusieurs fois, de préférence d'environ dix fois, la surface efficace (11) du piston de mesure.

9. Appareil selon l'une des revendications 6 à 8 qui comprend un support formé de quatre colonnes (1) avec une plaque supérieure (2) portant le cylindre de pression (3) et une plaque inférieure (4) portant le cylindre de mesure (5).

10. Appareil selon l'une des revendications 6 à 9 dans lequel le conduit d'arrivée d'air sous pression comporte une commande pour appliquer l'air alternativement à deux niveaux différents.

EP 0 275 825 B1